# EUROPÄISCHE PATENTSCHRIFT

(11) **EP 2 531 464 B1**
(45) Veröffentlichungstag und Bekanntmachung des Hinweises auf die Patenterteilung: **04.11.2015**
(21) Anmeldenummer: 11700391.3
(22) Anmeldetag: 13.01.2011
(51) Int. Cl.: C07C 2/08, C07C 11/02

(54) **VERFAHREN ZUR HERSTELLUNG LINEARER ALPHA-OLEFINE**
METHOD FOR PRODUCING LINEAR A-OLEFINS
PROCÉDÉ DE PRODUCTION D'A-OLÉFINES LINÉAIRES

(30) Priorität: 02.02.2010 DE 102010006589
(43) Veröffentlichungstag der Anmeldung: 12.12.2012
(73) Patentinhaber: Linde Aktiengesellschaft, 80331 München (DE)
(72) Erfinder: HOFMANN, Karl-Heinz, 82110 Germering (DE); ZANDER, Hans-Jörg, 81479 München (DE); WELLENHOFER, Anton, 82069 Hohenschäftlam (DE); MÜLLER, Wolfgang, 81245 München (DE); WÖHL, Anina, 81379 München (DE)
(86) Internationale Anmeldenummer: PCT/EP2011/000131
(87) Internationale Veröffentlichungsnummer: WO 2011/095273

(56) Entgegenhaltungen:
- WO-A1-2009/060343
- WO-A2-2009/060342
- FR-A- 1 419 732
- US-A- 3 655 812
- US-A- 4 155 946
- US-A1- 2009 203 946

## Beschreibung

Die Erfindung betrifft ein Verfahren zur Herstellung linearer α-Olefine durch Oligomerisierung von Ethylen in Anwesenheit eines organischen Lösungsmittels und eines homogenen Flüssigkatalysators in einem Reaktor.

Ein derartiges Verfahren zur Herstellung linearer α-Olefine durch Oligomerisierung von Ethylen ist beispielsweise in der DE 4338414 offenbart. Nach dem Stand der Technik findet die Oligomerisation in der Flüssigphase im unteren Teil eines Oligomerisierungsreaktors statt. Da die Reaktion exotherm ist und eine zu hohe Reaktionstemperatur zu einer Verschlechterung der Produktqualität führen würde, muss die Reaktionswärme abgeführt werden. Nach dem Stand der Technik erfolgt dies durch einen Kühlkreislauf mittels direkter Kühlung und gasförmigem Ethylen als Kältemittel. Gasförmiges Ethylen aus dem Ethylenkreislauf wird in den Reaktor geführt und in der Flüssigphase gelöst. Dadurch wird die für die Oligomisierungsreaktion benötigte Ethylenkonzentration aufrechterhalten. Mit dem Überschuss an Ethylen wird die Reaktionstemperatur gesteuert. Da die Reaktion stark exotherm ist, ist für die Abführung der Reaktionswärme, d.h. zur direkten Kühlung der Reaktion, eine große Menge gasförmigen Ethylens notwendig. Lediglich eine kleine Menge des Ethyleneinsatzes reagiert in der eigentlichen Oligomisierungsreaktion.

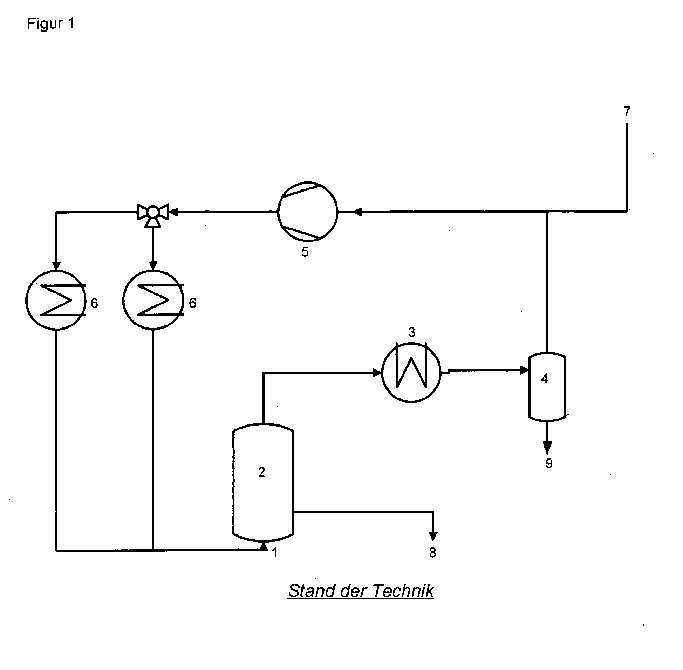

Der Stand der Technik soll nun anhand der Figur 1 näher erläutert werden. Das gasförmige Ethylen 1 wird dem Bodenbereich des Oligomerisierungsreaktors 2 zugeführt. Im Reaktor 2 befindet sich ein organisches Lösungsmittel mit einem homogenen Flüssigkatalysator. Das gasförmige Ethylen 1 durchquert dabei das Lösungsmittel mit dem Flüssigkatalysator, wobei ein geringer Teil des gasförmigen Ethylens zu linearen α-Olefinen oligomerisiert. Über den Kopf des Oligomerisierungsreaktors 2 verlässt den Reaktor ein Gemisch aus dem größten Teil des Ethylens, leichten α-Olefinen und, entsprechend dem thermodynamischen Gleichgewicht im Reaktor, etwas organisches Lösungsmittel. Dieses Gasgemisch wird in Kühler 3 abgekühlt und in den Abscheider 4 überführt. Die sich bei der Abkühlung bildende Flüssigphase 9 besteht hauptsächlich aus Lösungsmittel und leichten α-Olefinen und wird aus dem Sumpf des Abscheiders 4 abgezogen und in den Reaktor bzw. zur weiteren Trennung geführt (nicht dargestellt). Der Großteil des gasförmigen Ethylens verlässt den Abscheider 4 über Kopf und wird zusammen mit frischem Ethylen 7 in einen Kreislaufverdichter 5 geführt. In den nachfolgenden Wärmetauschern 6 wird das gasförmige Ethylen wieder auf die Einsatztemperatur, beispielsweise 10 °C angewärmt und als Einsatzstoff zurück in den Oligomerisierungsreaktor 2 geführt. Um die Einsatztemperatur des gasförmigen Ethylens zu regeln, sind hierbei zwei Wärmetauscher 6 nötig. Beide Wärmetauscher 6 werden auf eine konstante und voneinander verschiedene Temperatur geregelt. Die Einsatztemperatur des gasförmigen Ethylen 1 wird durch die relativen Anteile aus den beiden Wärmetauschern 6 reguliert, wobei die Gesamtmenge an gasförmigen Ethylen als Einsatz in den Reaktor konstant gehalten wird. Eine variable Einsatztemperatur des gasförmigen Ethylens 1 ist notwendig, um die Reaktortemperatur auch bei schwankenden Umsätzen und somit schwankender Wärmefreisetzung konstant zu halten. Das eigentliche Produkt 8 der Oligomerisierungsreaktion wird seitlich zusammen mit Lösemittel aus dem Oligomerisierungsreaktor 2 abgezogen. Das abgezogene Flüssiggemisch 8 wird anschließend in Lösungsmittel mit Flüssigkatalysator und das lineare α-Olefinprodukt getrennt. Das Lösungsmittel mit Flüssigkatalysator wird regeneriert und in den Oligomerisierungsreaktor zurückgeführt (nicht dargestellt). Die linearen α-Olefine werden in die einzelnen α-Olefine getrennt (nicht dargestellt).

Alternativ lässt sich die Temperatur des gasförmigen Ethylens 1, welches als Einsatz in den Reaktor 2 geführt wird, mit einem Wärmetauscher 6 regulieren. Hierbei muss jedoch die Temperatur des Wärmetauschers 6 variierbar sein.

Das geschilderte Verfahren nach dem Stand der Technik weist eine Reihe von Nachteilen auf. Um die Reaktionswärme des Oligomerisierungsreaktors abzuführen, muss eine große Menge gasförmigen Ethylens im Kreis gefahren werden. Entsprechend muss der Kreislaufverdichter sehr groß dimensioniert werden. Zum anderen ist die Steuerung der Reaktionstemperatur über die Einsatztemperatur des gasförmigen Ethylens mit den zwei Wärmetauschern oder einem regulierbaren Wärmetauscher aufwendig und kompliziert.

Zur Vermeidung dieser Nachteile wird in EP 1 748 038 vorgeschlagen, als Einsatzstoff für den Oligomerisierungsreaktor eine kleine Menge gasförmigen Ethylens und eine große Menge Inertgas zu verwenden. Als Inertgase werden hierbei hauptsächlich Kohlenwasserstoffe wie Methan, Ethan, Propan und Propylen sowie Wasserstoff vorgeschlagen. Auch hier muss eine große Gasmenge im Kreis geführt werden.

In EP 1 749 806 wird ein Verfahren zum Herstellen linearer α-Olefine durch Oligomerisieren von Ethylen offenbart, bei dem der Reaktorkopf mittels eines Kältemittels gekühlt wird, wobei die Temperatur im Reaktorkopf bei 15 bis 20°C gehalten wird und die Kühlung mittels Kondensator erfolgt, wobei als Kältemittel Propylen verwendet wird. Dabei wird Propylen am Kopf des Reaktors verflüssigt und im Bodenbereich des Reaktors verdampft. Dieses Verfahren hat den Nachteil, dass an den kalten Flächen des Kondensators verstärkt Belagsbildung, beispielsweise durch eingetragene Polymere, auftritt.

Weiterhin betrifft die WO 2009/060342A1 sowie WO2009/060343A1 ein Verfahren zur Polymerisation oder Oligomerisierung eines Kohlenwasserstoffs, wobei ein flüssiger Kohlenwasserstoffreaktant auf einem niedrigen Pegel einer Bulk-Flüssigphase zugeführt wird, die ein mit einem Katalysator gemischtes polymeres oder oligomeres Produkt aufweist. Die FR1419732A betrifft die Polymerisation von polymerisierbaren ungesättigten Verbindungen von Tetrahalo-Aluminaten. Die US3655812A betrifft die Polymerisierung eines ethylenhaltigen Gases in Gegenwart eines eine Titanverbindung aufweisenden Katalysators. Die US2009/203946A1 betrifft die Oligomerisierung niedrigerer Olefine ohne die Verwendung eines zugefügten Lösungsmittels. Schließlich betrifft die US4155946A die Dimerisierung niedriger Alpha-Olefine unter Verwendung eines Ziegler-Katalystors.

Der vorliegenden Erfindung liegt die Aufgabe zugrunde, ein Verfahren zur Herstellung linearer α-Olefine durch Oligomerisierung von Ethylen alternativ auszugestalten.

Die vorliegende Erfindung hat ferner die Aufgabe, die Menge Ethylen im Kreislauf zu reduzieren.

Des Weiteren soll die Belagsbildung in den Anlagenteilen reduziert werden.

Die vorliegenden Aufgaben werden durch die Merkmale des Anspruches 1 gelöst.

Verfahren zur Herstellung linearer α-Olefine durch Oligomerisierung von Ethylen (1, 1 a) in Anwesenheit eines organischen Lösungsmittels und eines homogenen Flüssigkatalysators in einem Reaktor (2), wobei Ethylen (1 a, 12) zumindest teilweise in flüssigem Aggregatzustand in den Reaktor (2) eingebracht wird, dadurch gekennzeichnet, dass gasförmig aus dem Reaktor (2) austretendes Ethylen nur teilweise kondensiert wird, wobei ein Zweiphasengemisch entsteht, das in einem Abscheider (4) getrennt wird, wobei die flüssige Phase direkt in den Reaktor (2) zurückgeführt wird, während die Gasphase nach Verdichtung gasförmig in den Reaktor (2) zurückgeführt wird.

Weitere vorteilhafte Ausgestaltungen der Erfindung werden in den Unteransprüchen angegeben.

Erfindungsgemäß wird das Ethylen zumindest teilweise in flüssigen Aggregatzustand in den Reaktor eingebracht. Dadurch erhöht sich die Kapazität zur Wärmeaufnahme des eingebrachten Ethylens deutlich. Bei dem Einbringen des Ethylens im flüssigen Aggregatzustand, kann das Ethylen deutlich mehr bei der Oligomerisierungsreaktion entstehende Wärme aufnehmen. Die Aufnahmekapazität von Wärme der Oligomerisierungsreaktion, wird dabei um den Betrag der Verdampfungswärme erhöht. Somit lässt sich die gleiche Wärmemenge der Oligomerisierungsreaktion durch eine deutlich geringere Menge an Ethylen aufnehmen. Dadurch kann die Menge im Ethylenkreislauf gegenüber dem Stand der Technik deutlich reduziert und die Temperaturregelung der Oligomerisierungsreaktion deutlich vereinfacht werden. Durch die geringere im Kreislauf geführte Menge reduziert sich auch die Belagsbildung in den jeweiligen Anlagenteilen, da auch deutlich weniger potentielle Belagsbildner im Kreis geführt werden. Durch die deutlich kleinere Menge an Ethylen, die im Kreis geführt wird, wird auch die Wahrscheinlichkeit von mitgerissenen Tröpfchen aus der ZweiPhasen-Schicht des Reaktors minimiert. Dadurch gelangen deutlich weniger potentielle Belagsbildner vom Reaktor in den Kreislauf. Zusätzlich erlaubt eine Zufuhr in der flüssigen Phase eine gute Vermischung des Ethylens mit dem in der flüssigen Phase vorliegenden Katalysatormaterial. Daher läuft die Oligomerisierungsreaktion mit unverminderter Ausbeute ab.

In einer vorteilhaften Ausgestaltung der Erfindung wird zusätzlich ein verflüssigtes Inertgas in den Reaktor eingebracht. Als Inertgas wird im Rahmen der Anmeldung jedes Gas verstanden, welches sich bezüglich der im Reaktor stattfindenden Reaktionen inert verhält. Bevorzugt werden dabei als verflüssigtes Inertgas Kohlenwasserstoffe, bevorzugt Propylen, Propan und/oder Kohlenwasserstoffe mit vier Kohlenstoffatomen, verwendet. In dieser Ausgestaltung der Erfindung wird zusätzlich zu Ethylen ein verflüssigtes Inertgas als Kälteträger in den Reaktor eingebracht. Das verflüssigte Inertgas verdampft im Reaktor und wird zusammen mit dem verdampften Ethylen wieder kondensiert und als Einsatzstoff in den Reaktor zurückgeführt. Das verflüssigte Inertgas wird dabei derart gewählt, dass es leicht verdampfbar und bei vertretbaren Temperaturen kondensierbar ist. Die genannten Inertgase sind hierbei ein guter Kompromiss aus leichter Verdampfbarkeit bei Reaktionsbedingungen und Kondensierbarkeit bei Kühlmitteltemperatur. Zusätzlich hat sich überraschender Weise gezeigt, dass Ethylen zusammen mit den genannten Inertgasen deutlich einfacher kondensiert werden kann als Ethylen allein. Der Energieaufwand für die Verflüssigung sinkt daher in dieser Ausgestaltung der Erfindung weiter.

In einer Ausgestaltung der Erfindung weist der Reaktor ein mechanisches Rührwerk, bevorzugt einen Gaseintragsrührer, besonders bevorzugt einen Hohlwellen-Eintragsrührer, auf. Durch das mechanische Rührwerk wird die Durchmischung der Gasphase der flüssigen Phase und dem flüssigen Katalysatormaterial deutlich verbessert. Bei der Zufuhr des Ethylens über das mechanische Rührwerk werden keine weiteren Einbauten benötigt und die Durchmischung wird deutlich effizienter gestaltet. Besonders die Verwendung eines Hohlwellen-Eintragsrührers ist zweckmäßig. Der Hohlwellen-Eintragsrührers saugt aus der Gasphase des Reaktors an, wodurch die Durchmischung im Reaktor weiter verbessert wird.

Das aus dem Reaktor gasförmig austretende Ethylen oder Ethylen und Inertgas wird nur teilweise kondensiert. Die Kondensation wird derart dimensioniert, dass nicht der komplette Gasstrom aus dem Reaktor kondensiert wird. Es entsteht ein Zweiphasengemisch. Das Zweiphasengemisch wird in einem Abscheider getrennt und die flüssige Phase aus Ethylen oder Ethylen und Inertgas direkt in den Reaktor zurückgeführt wird, während die Gasphase nach Verdichtung gasförmig in den Reaktor zurückgeführt wird. Der apparative Aufwand der Verdichtung gegenüber dem Stand der Technik wird dadurch deutlich reduziert.

Zusätzlich führt die zusätzliche Zufuhr in der Gasphase zu einer besseren Vermischung des Reaktorinhaltes im Vergleich zu Zurückführung einer reinen Flüssigphase. Das Gas verdrängt beim Reaktoreintritt die Flüssigphase und die entstehende Blasenbildung vergrößert die Turbulenz und somit die Durchmischung im Reaktor.

Vorteilhafterweise erfolgt die Steuerung der Temperaturregelung des Reaktors durch die Steuerung des Volumenstromes der zugeführten flüssigen Phase. Bei der Verwendung eines Kreislaufes mit einem flüssigen Kühlmittel, d.h. mit Ethylen in der flüssigen Phase oder Ethylen und ein Inertgas in der flüssigen Phase, kann die Reaktionstemperatur im Oligomerisierungsreaktor durch die Steuerung des Volumenstroms des Einsatzes des flüssigen Kühlmittels geregelt werden. Die Temperaturregelung durch die Steuerung des Volumenstromes ist dabei deutlich einfacher als eine Regelung der Einsatztemperatur. Daher kann in dieser Ausgestaltung der Erfindung auf die einen Wärmetauscher zur Regelung der Einsatztemperatur nach dem Stand der Technik verzichtet werden.

Mit der vorliegenden Erfindung gelingt es insbesondere, den apparativen Aufwand bei der Durchführung eines Verfahrens zur Herstellung von linearen α-Olefinen durch Oligomerisierung von Ethylen deutlich zu verringern. Durch die Zufuhr von Ethylen in der flüssigen Phase in den Oligomerisierungsreaktor wird die benötigte Kühlmittelmenge deutlich reduziert. Dadurch kann der Kreislaufverdichter für den Kühlkreislauf auf eine deutlich kleinere Menge ausgelegt und durch eine einfache Kreislaufpumpe ersetzt werden. Zusätzlich fällt bei Zuführung des Ethylens in der flüssigen Phase ein Wärmetauscher zur Regulierung der Einsatztemperatur weg bzw. wird die Regelung der Eintrittstemperatur des Ethylens deutlich vereinfacht. Damit werden die Investitionskostens eines derartigen Verfahrens gegenüber dem Stand der Technik deutlich reduziert. Weiterhin werden weniger Belagsbildner im Kreis geführt, so dass das Risiko der Belagsbildung und die damit verbundenen Reinigungen reduziert werden. Die vorliegende Erfindung bildet ein alternatives Verfahren zum Stand der Technik zur Herstellung linearer α-Olefine durch Oligomerisierung von Ethylen.

Im Folgenden soll die Erfindung anhand eines Vergleiches eines Ausführungsbeispieles der Erfindung mit dem Stand der Technik näher erläutert werden.

Es zeigen:
- Figur 1: ein Verfahren zur Herstellung von linearen α-Olefinen mittels Oligomerisierung nach dem Stand der Technik
- Figur 2: eine nicht-erfinderische Ausgestaltung
- Figur 3: eine Ausgestaltung der Erfindung

Figur 1 zeigt ein Verfahren zur Herstellung von linearen α-Olefinen nach dem Stand der Technik. Das in Figur 1 gezeigte Verfahren nach dem Stand der Technik verwendet Ethylen 1 in der gasförmigen Phase und wurde bereits im einleitenden Teil der Beschreibung erläutert.

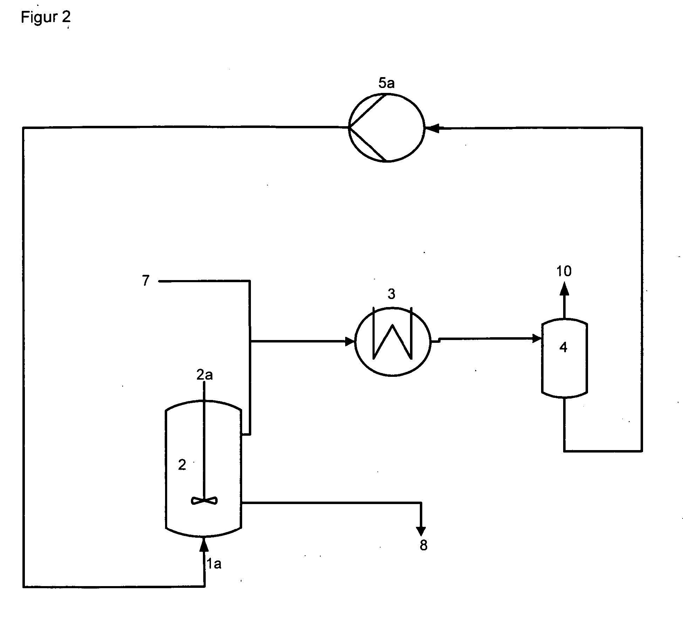

Figur 2 zeigt eine nicht-erfinderische Ausgestaltung in der Ethylen 1a in der flüssigen Phase in den Oligomerisierungsreaktor 2 geführt wird. Der Oligomerisierungsreaktor 2 weist ein mechanisches Rührwerk 2a auf, um eine optimale Vermischung des flüssigen Ethylens und des Katalysators in der Flüssigphase zu gewährleisten. Vom Kopf des Oligomerisierungsreaktors 2 wird verdampftes Ethylen zusammen mit leichten α-Olefinen und einem geringen Anteil des organischen Lösungsmittels abgezogen. Das abgezogene Gasgemisch vom Kopf des Reaktors 2 wird zusammen mit gasförmigem Frischethylen 7 mittels Wärmetauscher 3 und Abscheider 4 kondensiert. Die aus dem Abscheider 4 abgezogene Flüssigphase wird mittels Kreislaufpumpe 5a als flüssiger Ethyleneinsatz 1 a zurück in den Oligomerisierungsreaktor 2 geführt. Falls die Kondensation des Ethylens nicht vollständig erfolgt ist, wird die überschüssige Gasphase 10 vom Kopf des Abscheiders abgezogen. Durch den Abscheider 4 wird sichergestellt, dass kein Zweiphasengemisch in die Kreislaufpumpe 5a gelangt. Die flüssigen Produkte der Oligomerisierungsreaktion werden seitlich vom Boden des Reaktors 2 abgezogen 8.

Die Menge Ethylen im Kühlkreislauf hat sich gegenüber in Figur 1 gezeigten Stand der Technik dabei deutlich verringert. In beiden Fällen wurde versuchsweise die Oligomerisierungsreaktion bei einem Druck von ca. 30 bar und einer Temperatur von ca. 60 °C durchgeführt. In beiden Fällen wurden 10 Tonnen pro Stunde Flüssigprodukt 8 abgezogen und entsprechend 10 Tonnen pro Stunde gasförmiges Frischethylen 7 zugesetzt. Im Stand der Technik werden dabei mittels Kreislaufverdichter 200 Tonnen pro Stunde Ethylen zur Kühlung im Kreis gefahren. Dies entspricht einer Ethylenmenge von 5.000 Kubikmeter pro Stunde. Gemäß dem in Figur 2 gezeigten Ausführungsbeispiel der Erfindung werden im Kühlkreislauf lediglich 47 Tonnen pro Stunde Ethylen im Kreis gefahren. Dies entspricht 1200 Kubikmeter pro Stunde Ethylen in der flüssigen Phase. Dadurch kann eine einfache Kreislaufpumpe 5a im Gegensatz zum Kreislaufverdichter 5 nach dem Stand der Technik verwendet werden. Die beiden Wärmetauscher 6 nach dem Stand der Technik entfallen in diesem Ausführungsbeispiel der Erfindung völlig. Die Einsatztemperatur des Reaktors wird durch die Regulierung des Volumenstromes des flüssigen Ethylens 1 a kontrolliert.

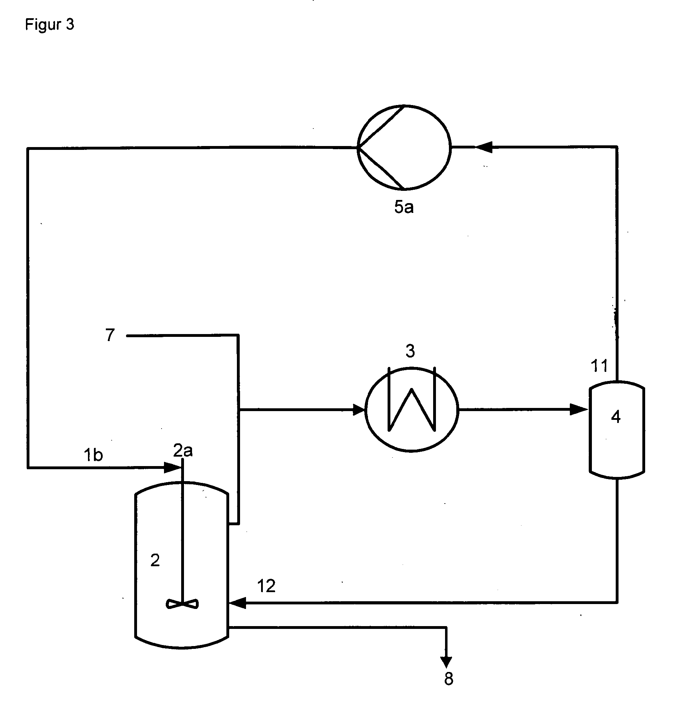

Figur 3 zeigt eine Ausgestaltung der Erfindung. In dieser Ausgestaltung der Erfindung wird das gasförmig aus dem Reaktor austretende Ethylen in dem Wärmetauscher 3 nicht vollständig kondensiert. Das entstandene Zweiphasengemisch wird im Abscheider 4 getrennt. Die gasförmige Phase 11 wird verdichtet und gasförmig in den Reaktor 2 zurückgeführt. Dabei wird das gasförmige Ethylen 1 b über einen Hohlwellen-Gaseintragsrührer 2a in den Reaktor 2 eingebracht. Die flüssige Phase 12 aus dem Abscheider 4 wird direkt in den Reaktor 2 zurückgeführt.

## Patentansprüche

1. Verfahren zur Herstellung linearer α-Olefine durch Oligomerisierung von Ethylen (1, 1 a) in Anwesenheit eines organischen Lösungsmittels und eines homogenen Flüssigkatalysators in einem Reaktor (2), wobei Ethylen (1a, 12) zumindest teilweise in flüssigem Aggregatzustand in den Reaktor (2) eingebracht wird,
**dadurch gekennzeichnet,**
**dass** gasförmig aus dem Reaktor (2) austretendes Ethylen nur teilweise kondensiert wird, wobei ein Zweiphasengemisch entsteht, das in einem Abscheider (4) getrennt wird, wobei die flüssige Phase direkt in den Reaktor (2) zurückgeführt wird, während die Gasphase nach Verdichtung gasförmig in den Reaktor (2) zurückgeführt wird.

2. Verfahren nach Anspruch 1,
**dadurch gekennzeichnet,**
**dass** zusätzlich ein verflüssigtes Inertgas in den Reaktor (2) eingebracht wird.

3. Verfahren nach Anspruch 2, **dadurch gekennzeichnet, dass** als verflüssigtes Inertgas Kohlenwasserstoffe, bevorzugt Propylen, Propan und/oder Kohlenwasserstoffe mit vier Kohlenstoffatomen, verwendet werden.

4. Verfahren nach einem der Ansprüche 1 bis 3, **dadurch gekennzeichnet, dass** der Reaktor (2) ein mechanisches Rührwerk (2a), bevorzugt einen Gaseintragsrührer, besonders bevorzugt einen Hohlwellen-Eintragsrührer, aufweist.

5. Verfahren nach einem der Ansprüche 1 bis 4, **dadurch gekennzeichnet, dass** die Temperaturregelung des Reaktors (2) durch die Steuerung des Volumenstromes der zugeführten flüssigen Phase (1a, 12) erfolgt.

## Claims

1. Process for preparing linear α-olefins by oligomerizing ethylene (1, 1a) in the presence of an organic solvent and of a homogeneous liquid catalyst in a reactor (2), wherein ethylene (1 a, 12) is introduced into the reactor (2) at least partly in the liquid state,
**characterized in that**
ethylene leaving the reactor (2) in gaseous form is only partly condensed, forming a biphasic mixture which is separated in a separator (4), the liquid phase being recycled directly into the reactor (2), while the gas phase, after compression, is recycled into the reactor (2) in gaseous form.

2. Process according to Claim 1, **characterized in that** a liquefied inert gas is additionally introduced into the reactor (2).

3. Process according to Claim 2, **characterized in that** the liquefied inert gas used comprises hydrocarbons, preferably propylene, propane and/or hydrocarbons having four carbon atoms.

4. Process according to any of Claims 1 to 3, **characterized in that** the reactor (2) has a mechanical stirrer (2a), preferably a gas-introducing stirrer, more preferably a hollow-shaft introducing stirrer.

5. Process according to any of Claims 1 to 4, **characterized in that** the temperature of the reactor (2) is regulated by the control of the volume flow of the liquid phase supplied (1 a, 12).

## Revendications

1. Procédé de fabrication d'α-oléfines linéaires par oligomérisation d'éthylène (1, 1a) en présence d'un solvant organique et d'un catalyseur liquide homogène dans un réacteur (2), l'éthylène (1a, 12) étant introduit dans le réacteur (2) au moins en partie à un état d'agrégation liquide, **caractérisé en ce que** l'éthylène sortant sous forme gazeuse du réacteur (2) n'est condensé qu'en partie, un mélange biphasé se formant, qui est séparé dans un séparateur (4), la phase liquide étant recyclée directement dans le réacteur (2), tandis que la phase gazeuse est recyclée dans le réacteur (2) sous forme gazeuse après compression.

2. Procédé selon la revendication 1, **caractérisé en ce qu'**un gaz inerte liquéfié est en outre introduit dans le réacteur (2).

3. Procédé selon la revendication 2, **caractérisé en ce que** des hydrocarbures, de préférence le propylène, le propane et/ou des hydrocarbures contenant quatre atomes de carbone sont utilisés en tant que gaz inerte liquéfié.

4. Procédé selon l'une quelconque des revendications 1 à 3, **caractérisé en ce que** le réacteur (2) comprend un agitateur mécanique (2a), de préférence un agitateur d'apport de gaz, de manière particulièrement préférée un agitateur d'apport à arbre creux.

5. Procédé selon l'une quelconque des revendications 1 à 4, **caractérisé en ce que** la régulation de température du réacteur (2) a lieu par ajustement du débit volumique de la phase liquide (1a, 12) introduite.
